# EUROPEAN PATENT SPECIFICATION

(11) **EP 4 138 543 B1**
(45) Date of publication and mention of the grant of the patent: **02.12.2026**
(21) Application number: 21793117.9
(22) Date of filing: 21.04.2021
(51) Int. Cl.: A01H 5/00, A01H 9/00, C07K 14/415, C12N 15/11

(54) **SOYBEAN PROMOTERS AND USES THEREOF**
SOJABOHNENPROMOTOREN UND VERWENDUNGEN DAVON
PROMOTEURS DE SOJA ET LEURS UTILISATIONS

(30) Priority: 23.04.2020 US 202063014232 P
(43) Date of publication of application: 01.03.2023
(73) Proprietor: Syngenta Crop Protection AG, 4058 Basel (CH)
(72) Inventor: CHEN, Zhongying, Durham, NC 27709 (US); ZHOU, Nan, Durham, NC 27709 (US); CLARKE V, Joseph, Dallas, Durham, NC 27709 (US); QUE, Qiudeng, Durham, NC 27709 (US)
(74) Representative: SYNGENTA IP
(86) International application number: PCT/US2021/028283
(87) International publication number: WO 2021/216630

(56) References cited:
- WO-A1-2014/025858
- WO-A1-2022/035649
- WO-A2-2014/150449
- US-A1- 2004 031 072
- US-A1- 2011 296 552

## Description

### RELATED APPLICATIONS

This application claims priority from provisional application 63/014,232 filed April 23, 2021.

### FIELD OF THE INVENTION

The present invention relates to promoters that are useful for expression cassettes in plants, such as soybean.

### SEQUENCE LISTING

This application is accompanied by a sequence listing entitled 82117-WO_sequence_listing.txt, created April 20, 2021, which is approximately 148 kb in size.

### BACKGROUND

Genetically modified plants are an important source of desirable traits, such as insect resistance and herbicide tolerance. In general, to create such traits, one or more nucleic acids are introduced into a plant containing expression cassettes that express one or more coding sequences for one or more traits. Such expression cassettes generally contain a promoter to control expression of each coding sequence. For certain traits, such as insect resistance and herbicide tolerance, it may be desirable to use promoters with medium to high level constitutive expression. The choices for such promoters remain limited. There remains a need for additional sequences for promoters that drive gene expression for robust protein production, ideally in all or most soybean tissues.

### SUMMARY

Provided herein are regulatory elements, such as promoters and terminators, obtained or derived from *Glycine* species, e.g., *Glycine max.* Such regulatory elements are useful for constructing expression cassettes for expression of a coding sequence of interest, such as expression of a coding sequence for a trait of interest in a plant. As described herein, several promoter sequences were tested and shown to be capable of expressing proteins in one or more tissues of interest in soybean. Accordingly, aspects of the disclosure relate to such promoters, and their use in expression cassettes, vectors, and transgenic plant and plant cells.

The present invention is defined by the appended claims.

In some aspects, the disclosure provides an expression cassette comprising a nucleotide sequence having at least 90% identity (e.g., at least 91%, at least 92%, at least 93%, at least 94%, at least 95%, at least 96%, at least 97%, at least 98%, at least 99%, or 100% identity) with one or more of SEQ ID NOs: 1-57, wherein the nucleotide sequence is operably linked to a heterologous nucleotide sequence. In some aspects, the disclosure provides an expression cassette comprising a nucleotide sequence comprising one or more of SEQ ID NOs: 1-57, or a biologically active fragment thereof (e.g., a fragment of at least 100, at least 200, at least 300, at least 400, at least 500, at least 600, at least 700, at least 800, at least 900 or at least 1000 contiguous nucleotides of one or more of SEQ ID NOs: 1-57), wherein the nucleotide sequence is operably linked to a heterologous nucleotide sequence. In some embodiments, the nucleotide sequence comprises any one of SEQ ID NOs: 1-5,7, 20-22, and 44-46. In some embodiments, the heterologous nucleotide sequence is a nucleic acid of interest that encodes an RNA or protein of interest. In some embodiments, the RNA or protein of interest is capable of conferring upon a plant a desired characteristic such as antibiotic resistance, virus resistance, insect resistance, disease resistance, resistance to other pests, herbicide tolerance, improved nutritional value, improved performance in an industrial process or altered reproductive capability. In some embodiments, the heterologous nucleotide sequence encodes a selectable marker or wherein expression cassette further comprises a selectable marker.

Other aspects of the disclosure provide a vector comprising the expression cassette of any one of the above-mentioned embodiments or any other embodiments provided herein. In some embodiments, the vector is a plasmid, virus, or Agrobacterium cell.

Other aspects of the disclosure provide plant cell comprising the expression cassette or vector of any one of the above-mentioned embodiments or any other embodiments provided herein. In some embodiments, the plant cell is a dicot cell. In some embodiments, the plant cell is a Glycine max cell.

Other aspects of the disclosure provide a transgenic plant comprising the plant cell of any one of the above-mentioned embodiments or any other embodiments provided herein. In some embodiments, the plant is a dicot. In some embodiments, the plant is a Glycine max plant.

Other aspects of the disclosure provide a seed from the transgenic plant of any one of the above-mentioned embodiments or any other embodiments provided herein.

Yet other aspects of the disclosure provide a method, comprising introducing the expression cassette or vector of any one of the above-mentioned embodiments or any other embodiments provided herein into a plant or plant cell. In some embodiments, the method further comprises placing the plant or plant cell under conditions whereby an RNA or protein of interest and/or a selectable marker is expressed from the expression cassette or vector. In some embodiments, the method further comprises crossing the plant to a second plant or self-crossing the plant to produce a progeny plant. In some embodiments, the disclosure provides a transgenic plant, or a plant part thereof, produced by the method of any one of the above-mentioned embodiments or any other embodiments provided herein. In some embodiments, the plant is a dicot. In some embodiments, the plant is a Glycine max plant.

### BRIEF DESCRIPTION OF THE DRAWINGS

Fig. 1 is a schematic of the gene structure of the highly expressed GmADF family member (Glyma09g02240).
Fig. 2 is a graph showing the relative expression level of promoter activity of different GmADF sequences vs CMP promoter activity in bombarded immature cotyledons (CMP promoter activity is normalized to 1.00).
Fig. 3 is a schematic of the gene structure of the highly expressed prGmADF-01 (SEQ ID NO: 1) and prGmADF-02 (SEQ ID NO: 5) promoter sequences.
Fig. 4 is a graph showing the average gene expression of candidate promoters. Errors bars represent standard deviation.
Fig. 5 is a graph showing the relative promoter activities compared to control promoter prCMP. The error bars represent the standard deviation for p01, 09, 10, 11, 12, 14, 28, 29, 31, and 37, but represent distance from the mean for p02, 25, 26, 27, 30, 32, and 35. Experiments for p13, 15, 33, 34, and 36 were not repeated, but similar results were observed when testing alternate variants.
Fig. 6 is a graph showing relative activity of promoter variants. The upper panel shows the length and nature (promoter, 5'UTR, CDS, and intron) of covered region for each promoter variant. The lower panel shows relative promoter activities for each variant in the assay.
Figs. 7A-7C are graphs showing promoter activities in driving selectable marker gene expression for stable transformation in tobacco and soybean. Promoter activities were reflected by the transformation frequency (TF) in tobacco (Fig. 7A) and soybean (Figs. 7B and 7C) Numbers in parentheses are construct numbers from Table 4.

### BRIEF DESCRIPTION OF THE SEQUENCES IN THE SEQUENCE LISTING

SEQ ID NO: 1 is GmADF1 regulatory sequences comprising GmADF promoter sequences, 5'-untranslated region (5'-UTR) and the 1st intron.
SEQ ID NO: 2 is GmADF1 regulatory sequences comprising GmADF promoter sequences, and 5'-untranslated region (5'-UTR) and the 1st intron.
SEQ ID NO: 3 is GmADF1 regulatory sequences comprising GmADF promoter sequences, and 5'-untranslated region (5'-UTR).
SEQ ID NO: 4 is GmADF1 regulatory sequences comprising GmADF promoter sequences, and 5'-untranslated region (5'-UTR).
SEQ ID NO: 5 is GmADF1 regulatory sequences comprising GmADF promoter sequences, and 5'-untranslated region (5'-UTR) and the 1st intron that have been modified as described in Example 3.
SEQ ID NO: 6 is a p01 promoter sequence.
SEQ ID NO: 7 is a p10 promoter sequence.
SEQ ID NO: 8 is a p11 promoter sequence.
SEQ ID NO: 9 is a p12 promoter sequence.
SEQ ID NO: 10 is a p14 promoter sequence.
SEQ ID NO: 11 is a p28 promoter sequence.
SEQ ID NO: 12 is a p30 promoter sequence.
SEQ ID NO: 13 is a p31 promoter sequence.
SEQ ID NO: 14 is a p32 promoter sequence.
SEQ ID NO: 15 is a p33 promoter sequence.
SEQ ID NO: 16 is a p35 promoter sequence.
SEQ ID NO: 17 is a p36 promoter sequence.
SEQ ID NO: 18 is a p37 promoter sequence.
SEQ ID NO: 19 is a p01 version 2 promoter sequence.
SEQ ID NO: 20 is a p10 version 2 promoter sequence.
SEQ ID NO: 21 is a p10 version 3 promoter sequence.
SEQ ID NO: 22 is a p10 version 4 promoter sequence.
SEQ ID NO: 23 is a p11 version 2 promoter sequence.
SEQ ID NO: 24 is a p11 version 3 promoter sequence.
SEQ ID NO: 25 is a p12 version 2 promoter sequence.
SEQ ID NO: 26 is a p12 version 3 promoter sequence.
SEQ ID NO: 27 is a p14 version 2 promoter sequence.
SEQ ID NO: 28 is a p31 version 2 promoter sequence.
SEQ ID NO: 29 is a p31 version 3 promoter sequence.
SEQ ID NO: 30 is a p32 version 2 promoter sequence.
SEQ ID NO: 31 is a p33 version 2 promoter sequence.
SEQ ID NO: 32 is a p33 version 3 promoter sequence.
SEQ ID NO: 33 is a p33 version 4 promoter sequence.
SEQ ID NO: 34 is a p35 version 2 promoter sequence.
SEQ ID NO: 35 is a p35 version 3 promoter sequence.
SEQ ID NO: 36 is a p35 version 4 promoter sequence.
SEQ ID NO: 37 is a p36 version 2 promoter sequence.
SEQ ID NO: 38 is a p36 version 3 promoter sequence.
SEQ ID NO: 39 is a p36 version 4 promoter sequence.
SEQ ID NO: 40 is a p37 version 2 promoter sequence.
SEQ ID NO: 41 is a p37 version 3 promoter sequence.
SEQ ID NO: 42 is a p01 version 3 promoter sequence.
SEQ ID NO: 43 is a prGmPIP-02 promoter sequence.
SEQ ID NO: 44 is a prGmADF-02 promoter sequence.
SEQ ID NO: 45 is a prGmADF-03 promoter sequence.
SEQ ID NO: 46 is a prGmADF-04 promoter sequence.
SEQ ID NO: 47 is a prGmCyn-02 promoter sequence.
SEQ ID NO: 48 is a prGmCypCMP-01 promoter sequence.
SEQ ID NO: 49 is a prGmCypCMP-02 promoter sequence.
SEQ ID NO: 50 is a prGmCypCMP-03 promoter sequence.
SEQ ID NO: 51 is a p28 version 2 promoter sequence.
SEQ ID NO: 52 is a p28 version 3 promoter sequence.
SEQ ID NO: 53 is a prGmSAMS-02 promoter sequence.
SEQ ID NO: 54 is a prGmSAMS-03 promoter sequence.
SEQ ID NO: 55 is a prGmGAPDH2-01 promoter sequence.
SEQ ID NO: 56 is a prGmGAPDH3-01 promoter sequence.
SEQ ID NO: 57 is a prGmGSK3-01 promoter sequence.

### DEFINITIONS

Although the following terms are believed to be well understood by one of ordinary skill in the art, the following definitions are set forth to facilitate understanding of the presently disclosed subject matter.

All technical and scientific terms used herein, unless otherwise defined below, are intended to have the same meaning as commonly understood by one of ordinary skill in the art. References to techniques employed herein are intended to refer to the techniques as commonly understood in the art, including variations on those techniques or substitutions of equivalent techniques that would be apparent to one of skill in the art.As used herein, the terms "a" or "an" or "the" may refer to one or more than one, unless the context clearly and unequivocally indicates otherwise. For example, "an" endogenous nucleic acid can mean one endogenous nucleic acid or a plurality of endogenous nucleic acids.

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc. As used herein in the specification and in the claims, "or" should be understood to have the same meaning as "and/or" as defined above.

The term "about" is used herein to mean approximately, roughly, around, or in the region of. When the term "about" is used in conjunction with a numerical range, it modifies that range by extending the boundaries above and below the numerical values set forth. In general, the term "about" is used herein to modify a numerical value above and below the stated value by a variance of 20 percent, preferably 10 percent up or down (higher or lower). With regard to a temperature the term "about" means ± 1 °C, preferably ± 0.5°C. Where the term "about" is used in the context of this invention (e.g., in combinations with temperature or molecular weight values) the exact value (i.e., without "about") is preferred.

As used herein, a "biologically active fragment" refers to a fragment of a reference sequence that has activity that is substantially equivalent to (e.g., at least 90% equivalent to) or greater than the activity of a reference sequence. For example, a biologically active fragment of a reference promoter would be a fragment that is capable of driving expression of a coding sequence at a substantially equivalent or higher level compared to the reference promoter.

A "coding sequence" is a nucleic acid sequence that is transcribed into RNA such as mRNA, rRNA, tRNA, snRNA, sense RNA or antisense RNA. In some embodiments, the RNA is then translated in an organism to produce a protein.

An "enhancer" is a nucleotide sequence that can stimulate promoter activity and can be an innate element of the promoter or a heterologous element inserted to enhance the level or tissue specificity of a promoter. The primary sequence can be present on either strand of a double-stranded DNA molecule, and is capable of functioning even when placed either upstream or downstream from the promoter.

The term "expression" when used with reference to a polynucleotide, such as a gene, ORF or portion thereof, or a transgene in plants, refers to the process of converting genetic information encoded in a gene into RNA (e.g., mRNA, rRNA, tRNA, or snRNA) through "transcription" of the gene (i.e., via the enzymatic action of an RNA polymerase), and into protein where applicable (e.g., if a gene encodes a protein), through "translation" of mRNA. Gene expression can be regulated at many stages in the process. For example, in the case of antisense or dsRNA constructs, respectively, expression may refer to the transcription of the antisense RNA only or the dsRNA only. In embodiments, "expression" refers to the transcription and stable accumulation of sense (mRNA) or functional RNA. "Expression" may also refer to the production of protein.

"Expression cassette" as used herein means a nucleic acid molecule capable of directing expression of a particular polynucleotide or polynucleotides in an appropriate host cell, comprising a promoter operably linked to the polynucleotide or polynucleotides of interest which is/are operably linked to termination signals. It also typically comprises polynucleotides required for proper translation of the polynucleotide or polynucleotides of interest. The expression cassette may also comprise polynucleotides not necessary in the direct expression of a polynucleotide of interest but which are present due to convenient restriction sites for removal of the cassette from an expression vector. The expression cassette comprising the polynucleotide(s) of interest may be chimeric, meaning that at least one of its components is heterologous with respect to at least one of its other components. The expression cassette may also be one that is naturally occurring but has been obtained in a recombinant form useful for heterologous expression. Typically, however, the expression cassette is heterologous with respect to the host, i.e. the particular polynucleotide of the expression cassette does not occur naturally in the host cell and must have been introduced into the host cell or an ancestor of the host cell by a transformation process known in the art. The expression of the polynucleotide(s) in the expression cassette is generally under the control of a promoter. In the case of a multicellular organism, such as a plant, the promoter can also be specific or preferential to a particular tissue, or organ, or stage of development. An expression cassette, or fragment thereof, can also be referred to as "inserted polynucleotide" or "insertion polynucleotide" when transformed into a plant.

"Gene of interest" or 'nucleic acid of interest (NOI)" refers to any gene or NOI which, when transferred to a plant, confers upon the plant a desired characteristic such as antibiotic resistance, virus resistance, insect resistance, disease resistance, resistance to other pests, herbicide tolerance, improved nutritional value, improved performance in an industrial process or altered reproductive capability. The "gene of interest" or NOI may also be one that is transferred to plants for the production of commercially valuable enzymes or metabolites in the plant.

A "heterologous" nucleic acid sequence is a nucleic acid sequence not naturally associated with a host cell into which it is introduced, including non-naturally occurring multiple copies of a naturally occurring nucleic acid sequence. A nucleic acid sequence can also be heterologous to other nucleic acid sequences with which it may be associated, for example in a nucleic acid construct, such as e.g., an expression vector. As one nonlimiting example, a promoter may be present in a nucleic acid construct in combination with one or more regulatory element and/or coding sequences that do not naturally occur in association with that particular promoter, i.e., they are heterologous to the promoter.

An "isolated" nucleic acid molecule or nucleotide sequence or an "isolated" polypeptide is a nucleic acid molecule, nucleotide sequence or polypeptide that, by the hand of man, exists apart from its native environment and/or has a function that is different, modified, modulated and/or altered as compared to its function in its native environment and is therefore not a product of nature. An isolated nucleic acid molecule or isolated polypeptide may exist in a purified form or may exist in a non-native environment such as, for example, a recombinant host cell. Thus, for example, with respect to polynucleotides, the term isolated means that it is separated from the chromosome and/or cell in which it naturally occurs. A polynucleotide is also isolated if it is separated from the chromosome and/or cell in which it naturally occurs and is then inserted into a genetic context, a chromosome, a chromosome location, and/or a cell in which it does not naturally occur. The recombinant nucleic acid molecules and nucleotide sequences of the invention can be considered to be "isolated" as defined above.

Thus, an "isolated nucleic acid molecule" or "isolated nucleotide sequence" is a nucleic acid molecule or nucleotide sequence that is not immediately contiguous with nucleotide sequences with which it is immediately contiguous (one on the 5' end and one on the 3' end) in the naturally occurring genome of the organism from which it is derived. Accordingly, in one embodiment, an isolated nucleic acid includes some or all of the 5' non-coding (e.g., promoter) sequences that are immediately contiguous to a translation start site or transcription start site of a coding sequence. The term therefore includes, for example, a recombinant nucleic acid that is incorporated into a vector, into an autonomously replicating plasmid or virus, or into the genomic DNA of a prokaryote or eukaryote, or which exists as a separate molecule (e.g., a cDNA or a genomic DNA fragment produced by PCR or restriction endonuclease treatment), independent of other sequences. It also includes a recombinant nucleic acid that is part of a hybrid nucleic acid molecule encoding an additional polypeptide or peptide sequence. An "isolated nucleic acid molecule" or "isolated nucleotide sequence" can also include a nucleotide sequence derived from and inserted into the same natural, original cell type, but which is present in a non-natural state, e.g., present in a different copy number, and/or under the control of different regulatory sequences than that found in the native state of the nucleic acid molecule.

The term "isolated" can further refer to a nucleic acid molecule, nucleotide sequence, polypeptide, peptide or fragment that is substantially free of cellular material, viral material, and/or culture medium (e.g., when produced by recombinant DNA techniques), or chemical precursors or other chemicals (e.g., when chemically synthesized). Moreover, an "isolated fragment" is a fragment of a nucleic acid molecule, nucleotide sequence or polypeptide that is not naturally occurring as a fragment and would not be found as such in the natural state. "Isolated" does not necessarily mean that the preparation is technically pure (homogeneous), but it is sufficiently pure to provide the polypeptide or nucleic acid in a form in which it can be used for the intended purpose.

In representative embodiments of the invention, an "isolated" nucleic acid molecule, nucleotide sequence, and/or polypeptide is at least about 5%, 10%, 15%, 20%, 25%, 30%, 40%, 50%, 60%, 70%, 75%, 80%, 85%, 90%, 95%, 97%, 98%, 99% pure (w/w) or more. In other embodiments, an "isolated" nucleic acid, nucleotide sequence, and/or polypeptide indicates that at least about a 5-fold, 10-fold, 25-fold, 100-fold, 1000-fold, 10,000-fold, 100,000-fold or more enrichment of the nucleic acid (w/w) is achieved as compared with the starting material.

The term "introducing" or "introduce" in the context of a plant cell, plant and/or plant part means contacting a nucleic acid molecule with the plant, plant part, and/or plant cell in such a manner that the nucleic acid molecule gains access to the interior of the plant cell and/or a cell of the plant and/or plant part. Where more than one nucleic acid molecule is to be introduced these nucleic acid molecules can be assembled as part of a single polynucleotide or nucleic acid construct, or as separate polynucleotide or nucleic acid constructs, and can be located on the same or different nucleic acid constructs. Accordingly, these polynucleotides can be introduced into plant cells in a single transformation event, in separate transformation events, or, e.g., as part of a breeding protocol. Thus, the term "transformation" as used herein refers to the introduction of a heterologous nucleic acid into a cell. Transformation of a cell may be stable or transient. Thus, a transgenic plant cell, plant and/or plant part of the invention can be stably transformed or transiently transformed.

The terms "percent sequence identity" or "percent identity" are used interchangeably herein and is used herein refers to the percentage of identical nucleotides or amino acids in a linear polynucleotide or amino acid sequence of a reference ("query") sequence (or its complementary strand) as compared to a test ("subject") sequence (or its complementary strand) when the two sequences are optimally aligned. Optimal alignment of sequences for aligning a comparison window are known to those skilled in the art and may be conducted using known methods, e.g., using known software or computer programs such as the Smith and Waterman algorithm implemented in the EMBOSS-6.6.0 water tool using default matrix files EBLOSUM62 for protein, EDNAFULL for DNA with default gap penalties. EMBOSS-6.6.0 is available, e.g., from the following Bio-soft and Open-Bio such as at the following websites: en.bio-soft.net/format/emboss.html or emboss.open-bio.org/html/adm/ch01s01.html.

The terms "nucleic acid" or "polynucleotide" are used interchangeably herein and refer to any physical string of monomer units that can be corresponded to a string of nucleotides, including a polymer of nucleotides (e.g., a typical DNA polymer or polydeoxyribonucleotide or RNA polymer or polyribonucleotide), modified oligonucleotides (e.g., oligonucleotides comprising bases that are not typical to biological RNA or DNA, such as 2'-O-methylated oligonucleotides), and the like. In some embodiments, a nucleic acid or polynucleotide can be single-stranded, double-stranded, multi-stranded, or combinations thereof. Unless otherwise indicated, a particular nucleic acid or polynucleotide of the present invention optionally comprises or encodes complementary polynucleotides, in addition to any polynucleotide explicitly indicated. The nucleic acid can be present in a vector, such as in a cell, virus or plasmid.

"Operably linked" refers to the association of polynucleotides on a single nucleic acid fragment so that the function of one affects the function of the other. For example, a promoter is operably linked with a coding polynucleotide when it is capable of affecting the expression of that coding polynucleotide (i.e., that the coding polynucleotide is under the transcriptional control of the promoter). Coding polynucleotide in sense or antisense orientation can be operably linked to regulatory polynucleotides.

The term "plant" refers to any plant, particularly to agronomically useful plants (e.g. seed plants), and "plant cell" is a structural and physiological unit of the plant, which comprises a cell wall but may also refer to a protoplast. The plant cell may be in form of an isolated single cell or a cultured cell, or as a part of higher organized units such as for example, a plant tissue, or a plant organ differentiated into a structure that is present at any stage of a plant's development. A plant may be a monocotyledonous or dicotyledonous plant species.

A "plant cell" is a structural and physiological unit of a plant, comprising a protoplast and a cell wall. The plant cell may be in form of an isolated single cell or a cultured cell, or as a part of higher organized unit such as, for example, plant tissue, a plant organ, or a whole plant.

The term "plant part," as used herein, includes but is not limited to embryos, pollen, ovules, seeds, leaves, stems, shoots, flowers, branches, fruit, kernels, ears, cobs, husks, stalks, roots, root tips, anthers, plant cells including plant cells that are intact in plants and/or parts of plants, plant protoplasts, plant tissues, plant cell tissue cultures, plant calli, plant clumps, and the like. As used herein, "shoot" refers to the above ground parts including the leaves and stems. Further, as used herein, "plant cell" refers to a structural and physiological unit of the plant, which comprises a cell wall and also may refer to a protoplast. A plant cell of the present invention can be in the form of an isolated single cell or can be a cultured cell or can be a part of a higher-organized unit such as, for example, a plant tissue or a plant organ.

"Plant tissue" as used herein means a group of plant cells organized into a structural and functional unit. Any tissue of a plant in planta or in culture is included. This term includes, but is not limited to, whole plants, plant organs, plant seeds, tissue culture and any group of plant cells organized into structural and/or functional units. The use of this term in conjunction with, or in the absence of, any specific type of plant tissue as listed above or otherwise embraced by this definition is not intended to be exclusive of any other type of plant tissue.

The term "promoter," as used herein, refers to a polynucleotide, usually upstream (5') of the translation start site of a coding sequence, which controls the expression of the coding sequence by providing the recognition for RNA polymerase and other factors required for proper transcription. For example, a promoter may contain a region containing basal promoter elements recognized by RNA polymerase, a region containing the 5' untranslated region (UTR) of a coding sequence, and optionally an intron.

"Regulatory elements" and "regulatory sequences" are used interchangeably herein and refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences include enhancers, promoters, translational enhancer sequences, introns, terminators, and polyadenylation signal sequences. They include natural and synthetic sequences as well as sequences which may be a combination of synthetic and natural sequences. Regulatory sequences may determine expression level, the spatial and temporal pattern of expression and, for a subset of promoters, expression under inductive conditions (regulation by external factors such as light, temperature, chemicals and hormones). Regulatory sequences may be short regions of DNA sequence 6-100 base pairs that define the binding sites for trans-acting factors, such as transcription factors. Regulatory sequences may also be enhancers, longer regions of DNA sequence that can act from a distance from the core promoter region, sometimes over several kilobases from the core region. Regulatory sequence activity may be influenced by trans-acting factors including general transcription machinery, transcription factors and chromatin assembly factors.

A "terminator" is responsible for the termination of transcription beyond the coding region and correct mRNA polyadenylation. The termination region may be native with the transcriptional initiation region, may be native with the operably linked DNA sequence of interest, may be native with the plant host, or may be derived from another source (i.e., foreign or heterologous to the promoter, the DNA sequence of interest, the plant host, or any combination thereof). Appropriate transcriptional terminators are those that are known to function in plants and include the CAMV 35S terminator, the tml terminator, the nopaline synthase terminator and the pea rbcs E9 terminator. These can be used in both monocotyledons and dicotyledons. In addition, a gene's native transcription terminator may be used.

A "selectable marker" or "selectable marker gene" refers to a gene whose expression in a plant cell gives the cell a selective advantage. "Positive selection" refers to a transformed cell acquiring the ability to metabolize a substrate that it previously could not use or could not use efficiently, typically by being transformed with and expressing a positive selectable marker gene. This transformed cell thereby grows out of the mass of nontransformed tissue. Positive selection can be of many types from inactive forms of plant growth regulators that are then converted to active forms by the transferred enzyme to alternative carbohydrate sources that are not utilized efficiently by the nontransformed cells, for example mannose, which then become available upon transformation with an enzyme, for example phosphomannose isomerase, that allows them to be metabolized. Nontransformed cells either grow slowly in comparison to transformed cells or not at all. Other types of selection may be due to the cells transformed with the selectable marker gene gaining the ability to grow in presence of a negative selection agent, such as an antibiotic or an herbicide, compared to the ability to grow of non-transformed cells. A selective advantage possessed by a transformed cell may also be due to the loss of a previously possessed gene in what is called "negative selection". In this, a compound is added that is toxic only to cells that did not lose a specific gene (a negative selectable marker gene) present in the parent cell (typically a transgene).

Examples of selectable markers include, but are not limited to, genes that provide resistance or tolerance to antibiotics such as kanamycin (Dekeyser et al. 1989, Plant Phys 90: 217-23), spectinomycin (Svab and Maliga 1993, Plant Mol Biol 14: 197-205), streptomycin (Maliga et al. 1988, Mol Gen Genet 214: 456-459), hygromycin B (Waldron et al. 1985, Plant Mol Biol 5: 103-108), bleomycin (Hille et al. 1986, Plant Mol Biol 7: 171-176), sulphonamides (Guerineau et al. 1990, Plant Mol Biol 15: 127-136), streptothricin (Jelenska et al. 2000, Plant Cell Rep 19: 298-303), or chloramphenicol (De Block et al. 1984, EMBO J 3: 1681-1689). Other selectable markers include genes that provide resistance or tolerance to herbicides, such as the S4 and/or Hra mutations of acetolactate synthase (ALS) that confer resistance to herbicides including sulfonylureas, imidazolinones, triazolopyrimidines, and pyrimidinyl thiobenzoates; 5-enol-pyrovyl-shikimate-3-phosphate-synthase (EPSPS) genes, including but not limited to those described in U.S. Patent. Nos. 4,940,935,5,188,642, 5,633,435, 6,566,587, 7,674,598 (as well as all related applications) and the glyphosate N-acetyltransferase (GAT) which confers resistance to glyphosate (Castle et al. 2004, Science 304:1151-1154, and U.S. Patent Application Publication Nos. 20070004912, 20050246798, and 20050060767); BAR which confers resistance to glufosinate (see e.g., U.S. Patent Nos. 5,561,236); aryloxy alkanoate dioxygenase or AAD-1, AAD-12, or AAD-13 which confer resistance to 2,4-D; genes such as Pseudomonas HPPD which confer HPPD resistance; Sprotophorphyrinogen oxidase (PPO) mutants and variants, which confer resistance to peroxidizing herbicides including fomesafen, acifluorfen-sodium, oxyfluorfen, lactofen, fluthiacet-methyl, saflufenacil, flumioxazin, flumiclorac-pentyl, carfentrazone-ethyl, sulfentrazone,); and genes conferring resistance to dicamba, such as dicamba monoxygenase (Herman et al. 2005, J Biol Chem 280: 24759-24767 and U.S. Patent No. 7,812,224 and related applications and patents). Other examples of selectable markers can be found in Sundar and Sakthivel (2008, J Plant Physiology 165: 1698-1716).

Other selection systems include using drugs, metabolite analogs, metabolic intermediates, and enzymes for positive selection or conditional positive selection of transgenic plants. Examples include, but are not limited to, a gene encoding phosphomannose isomerase (PMI) where mannose is the selection agent, or a gene encoding xylose isomerase where D-xylose is the selection agent (Haldrup et al. 1998, Plant Mol Biol 37: 287-96). Finally, other selection systems may use hormone-free medium as the selection agent. One non-limiting example the maize homeobox gene *kn1*, whose ectopic expression results in a 3-fold increase in transformation efficiency (Luo et al. 2006, Plant Cell Rep 25: 403-409). Examples of various selectable markers and genes encoding them are disclosed in Miki and McHugh (J Biotechnol, 2004, 107: 193-232).

In some embodiments of the disclosure, the selectable marker may be plant derived. An example of a selectable marker which can be plant derived includes, but is not limited to, 5-enolpyruvylshikimate-3-phosphate synthase (EPSPS). The enzyme 5-enolpyruvylshikimate-3-phosphate synthase (EPSPS) catalyzes an essential step in the shikimate pathway common to aromatic amino acid biosynthesis in plants. The herbicide glyphosate inhibits EPSPS, thereby killing the plant. Transgenic glyphosate-tolerant plants can be created by the introduction of a modified EPSPS transgene which is not affected by glyphosate (for example, US Patent 6,040,497). Other examples of a modified plant EPSPS which can be used as a selectable marker in the presence of glyphosate includes a P106L mutant of rice EPSPS (Zhou et al 2006, Plant Physiol 140: 184-195) and a P106S mutation in goosegrass EPSPS (Baerson et al 2002, Plant Physiol 129: 1265-1275). Other sources of EPSPS which are not plant derived and can be used to confer glyphosate tolerance include but are not limited to an EPSPS P101S mutant from Salmonella typhimurium (Comai et al 1985, Nature 317: 741-744) and a mutated version of CP4 EPSPS from *Agrobacterium* sp. Strain CP4 (Funke et al 2006, PNAS 103: 13010-13015). Although the plant EPSPS gene is nuclear, the mature enzyme is localized in the chloroplast (Mousdale and Coggins 1985, Planta 163:241-249). EPSPS is synthesized as a preprotein containing a transit peptide, and the precursor is then transported into the chloroplast stroma and proteolytically processed to yield the mature enzyme (della-Cioppa et al. 1986, PNAS 83: 6873-6877). Therefore, to create a transgenic plant which has tolerance to glyphosate, a suitably mutated version of EPSPS which correctly translocates to the chloroplast could be introduced. Such a transgenic plant then has a native, genomic EPSPS gene as well as the mutated EPSPS transgene. Glyphosate could then be used as a selection agent during the transformation and regeneration process, whereby only those plants or plant tissue that are successfully transformed with the mutated EPSPS transgene survive.

The term "transformation" as used herein refers to the transfer of a nucleic acid into a host cell, preferably resulting in genetically stable integration, which includes integration into a chromosome and heritable extrachromosomal events. In some particular embodiments, the introduction into a plant, plant part and/or plant cell is via bacterial-mediated transformation, particle bombardment transformation (also called biolistic particle transformation), calcium-phosphate-mediated transformation, cyclodextrin-mediated transformation, electroporation, liposome-mediated transformation, nanoparticle-mediated transformation, polymer-mediated transformation, virus-mediated nucleic acid delivery, whisker-mediated nucleic acid delivery, microinjection, sonication, infiltration, polyethylene glycol-mediated transformation, protoplast transformation, or any other electrical, chemical, physical and/or biological mechanism that results in the introduction of a nucleic acid into the plant, plant part and/or cell thereof, or a combination thereof. General guides to various plant transformation methods known in the art include Miki et al. ("Procedures for Introducing Foreign DNA into Plants" in Methods in Plant Molecular Biology and Biotechnology, Glick, B. R. and Thompson, J. E., Eds. (CRC Press, Inc., Boca Raton, 1993), pages 67-88) and Rakowoczy-Trojanowska (2002, Cell Mol Biol Lett 7:849-858 (2002)).

Procedures for transforming plants are well known and routine in the art and are described throughout the literature. Non-limiting examples of methods for transformation of plants include transformation via bacterial-mediated nucleic acid delivery (*e.g*., via bacteria from the genus *Agrobacterium),* viral-mediated nucleic acid delivery, silicon carbide or nucleic acid whisker-mediated nucleic acid delivery, liposome mediated nucleic acid delivery, microinjection, microparticle bombardment, calcium-phosphate-mediated transformation, cyclodextrin-mediated transformation, electroporation, nanoparticle-mediated transformation,, sonication, infiltration, PEG-mediated nucleic acid uptake, as well as any other electrical, chemical, physical (mechanical) and/or biological mechanism that results in the introduction of nucleic acid into the plant cell, including any combination thereof. General guides to various plant transformation methods known in the art include Miki et al. ("Procedures for Introducing Foreign DNA into Plants" in Methods in Plant Molecular Biology and Biotechnology, Glick, B. R. and Thompson, J. E., Eds. (CRC Press, Inc., Boca Raton, 1993), pages 67-88) and Rakowoczy-Trojanowska (Cell Mol Biol Lett 7:849-858 (2002)).

*Agrobacterium-mediated* transformation is a commonly used method for transforming plants because of its high efficiency of transformation and because of its broad utility with many different species. *Agrobacterium-mediated* transformation typically involves transfer of the binary vector carrying the foreign DNA of interest to an appropriate *Agrobacterium* strain that may depend on the complement of vir genes carried by the host *Agrobacterium* strain either on a co-resident Ti plasmid or chromosomally (Uknes et al. 1993, Plant Cell 5:159-169). The transfer of the recombinant binary vector to *Agrobacterium* can be accomplished by a tri-parental mating procedure using *Escherichia coli* carrying the recombinant binary vector, a helper *E. coli* strain that carries a plasmid that is able to mobilize the recombinant binary vector to the target *Agrobacterium* strain. Alternatively, the recombinant binary vector can be transferred to *Agrobacterium* by nucleic acid transformation (Höfgen and Willmitzer 1988, Nucleic Acids Res 16:9877).

Transformation of a plant by recombinant *Agrobacterium* usually involves cocultivation of the *Agrobacterium* with explants from the plant and follows methods well known in the art. Transformed tissue is typically regenerated on selection medium carrying an antibiotic or herbicide resistance marker between the binary plasmid T-DNA borders.

Another method for transforming plants, plant parts and plant cells involves propelling inert or biologically active particles at plant tissues and cells. *See, e.g.,* US Patent Nos. 4,945,050; 5,036,006 and 5,100,792. Generally, this method involves propelling inert or biologically active particles at the plant cells under conditions effective to penetrate the outer surface of the cell and afford incorporation within the interior thereof. When inert particles are utilized, the vector can be introduced into the cell by coating the particles with the vector containing the nucleic acid of interest. Alternatively, a cell or cells can be surrounded by the vector so that the vector is carried into the cell by the wake of the particle. Biologically active particles (*e.g*., dried yeast cells, dried bacteria or a bacteriophage, each containing one or more nucleic acids sought to be introduced) also can be propelled into plant tissue.

Thus, in particular embodiments of the present invention, a plant cell can be transformed by any method known in the art and as described herein and intact plants can be regenerated from these transformed cells using any of a variety of known techniques. Plant regeneration from plant cells, plant tissue culture and/or cultured protoplasts is described, for example, in Evans et al. (Handbook of Plant Cell Cultures, Vol. 1, MacMilan Publishing Co. New York (1983)); and Vasil I. R. (ed.) (Cell Culture and Somatic Cell Genetics of Plants, Acad. Press, Orlando, Vol. I (1984), and Vol. II (1986)). Methods of selecting for transformed transgenic plants, plant cells and/or plant tissue culture are routine in the art and can be employed in the methods of the invention provided herein.

A "transgenic plant" is a plant having one or more plant cells that contain a heterologous DNA sequence.

As used herein, "vector" includes reference to a nucleic acid used in transfection of a host cell and into which can be inserted a polynucleotide. Vectors are often replicons. Expression vectors permit transcription of a nucleic acid inserted therein. "Vector" is defined to include, inter alia, any plasmid, cosmid, phage or Agrobacterium binary vector in double or single stranded linear or circular form which may or may not be self transmissible or mobilizable, and which can transform prokaryotic or eukaryotic host either by integration into the cellular genome or exist extrachromosomally (e.g. autonomous replicating plasmid with an origin of replication). Specifically included are shuttle vectors by which is meant a DNA vehicle capable, naturally or by design, of replication in two different host organisms, which may be selected from actinomycetes and related species, bacteria and eukaryotic (e.g. higher plant, mammalian, yeast or fungal cells).

### DETAILED DESCRIPTION

Aspects of the disclosure relate to regulatory elements, such as promoters and terminators, useful for expression of heterologous sequences in plants, such as soybean.

In some aspects, the disclosure provides a nucleotide sequence having at least 90% identity (e.g., at least 91%, at least 92%, at least 93%, at least 94%, at least 95%, at least 96%, at least 97%, at least 98%, at least 99%, or 100% identity) with one or more of SEQ ID NOs: 1-57. In some embodiments, the nucleotide sequence comprises one or more of SEQ ID NOs: SEQ ID NOs: 1-5,7, 20-22 and 44-46. In some embodiments, the nucleotide sequence comprises one of SEQ ID Nos: SEQ ID NOs: 1-5,7, 20-22 and 44-46. In some aspects, the disclosure provides a nucleotide sequence comprising a fragment, e.g., a biologically active fragment, of one or more of SEQ ID NOs: SEQ ID NOs: 1-57 (e.g., a fragment of at least 100, at least 200, at least 300, at least 400, at least 500, at least 600, at least 700, at least 800, at least 900 or at least 1000 contiguous nucleotides of one or more of SEQ ID NOs: 1-57). In some aspects, the disclosure provides a nucleotide sequence comprising a fragment, e.g., a biologically active fragment, of one or more of SEQ ID NOs: 1-57 (e.g., a fragment of at least 100, at least 200, at least 300, at least 400, at least 500, at least 600, at least 700, at least 800, at least 900 or at least 1000 contiguous nucleotides of one or more of SEQ ID NOs: 1-57).

In some aspects, the disclosure provides an expression cassette. In some embodiments, the expression cassette comprises a nucleotide sequence having at least 90% identity (e.g., at least 91%, at least 92%, at least 93%, at least 94%, at least 95%, at least 96%, at least 97%, at least 98%, at least 99%, or 100% identity) with one or more of SEQ ID NOs: 1-5,7, 20-22 and 44-46, wherein the nucleotide sequence is operably linked to a heterologous nucleotide sequence. In some embodiments, the expression cassette comprises a nucleotide sequence comprising one or more of SEQ ID NOs: SEQ ID NOs: 1-5,7, 20-22 and 44-46. In some embodiments, the expression cassette comprises a nucleotide sequence comprising one of SEQ ID NOs: SEQ ID NOs: 1-5,7, 20-22 and 44-46. In some embodiments, the expression cassette comprises a nucleotide sequence comprising a fragment, e.g., a biologically active fragment, of one or more of SEQ ID NOs: SEQ ID NOs: 1-5,7, 20-22 and 44-46 (e.g., a fragment of at least 100, at least 200, at least 300, at least 400, at least 500, at least 600, at least 700, at least 800, at least 900 or at least 1000 contiguous nucleotides of one or more of SEQ ID NOs: SEQ ID NOs: 1-5, 7, 20-22 and 44-46). In some embodiments, the expression cassette further comprises a selectable marker.

In some embodiments, the heterologous sequence is a nucleic acid of interest that encodes an RNA or protein of interest. In some embodiments, the RNA or protein of interest is capable of conferring upon a plant a desired characteristic such as antibiotic resistance, virus resistance, insect resistance, disease resistance, resistance to other pests, herbicide tolerance, improved nutritional value, improved performance in an industrial process or altered reproductive capability. In some embodiments, the RNA or protein of interest comprises a genome editing agent, e.g., a CRISPR/Cas agent (such as a Cas protein and/or guide RNA), a TALEN, a DNA-guided nuclease, a meganuclease, a recombinase, or a zinc finger nuclease. In some embodiments, the heterologous nucleotide sequence encodes a selectable marker.

In some embodiments, the expression cassette is comprised within a vector, such as a plasmid, virus, or Agrobacterium. In some embodiments, the expression cassette is comprised within a plant cell. In some embodiments, the plant cell is a dicot cell. In some embodiments, the plant cell is a *Glycine max* cell. In some embodiments, the *Glycine max* cell is an elite *Glycine max* cell.

In some embodiments, the expression cassette is comprised within a transgenic plant. In some embodiments, the plant is a dicot. In some embodiments, the plant is a *Glycine max* plant. In some embodiments, the *Glycine max* plant is an elite *Glycine max* plant.

In some embodiments, the disclosure provides a seed from a transgenic plant, e.g., a seed comprising the expression cassette.

In some embodiments, the disclosure provides a commodity product produced from a transgenic plant or part thereof, e.g., a commodity product comprising the expression casssette. In some embodiments, the commodity product is selected from the group consisting of whole or processed seeds, flour, protein isolates, concentrates, liquids, syrups, pastes, sauces or other food or product.

Other aspects of the disclosure relate to a method, e.g., a transformation method, comprising introducing an expression cassette or vector as described herein into a plant or plant cell through transformation. In some embodiments, the introducing comprises *Agrobacterium-mediated* transformation. In some embodiments, the introducing comprises particle bombardment. In some embodiments of the method, the method further comprises placing the plant or plant cell under conditions whereby an RNA or protein of interest and/or a selectable marker is expressed from the expression cassette or vector. In some embodiments, the conditions are appropriate growth or maintenance conditions for the plant or plant cell. In some embodiments of the method, the method further comprises crossing the plant to a second plant to produce a progeny plant. In some embodiments of the method, the method further comprises self-crossing the plant to produce a progeny plant. In some embodiments, the plant or plant cell is a dicot plant or plant cell. In some embodiments, the plant or plant cell is a *Glycine max* plant or plant cell. In some embodiments, the plant or plant cell is an elite *Glycine max* plant or plant cell. In some embodiments, the second plant is an elite *Glycine max* plant.

Hereinafter, the present invention will be described in detail by the following examples. However, the following examples are illustrative of the present invention.

### EXAMPLES

### Example 1: Identification of GmADF1 candidate promoter sequences

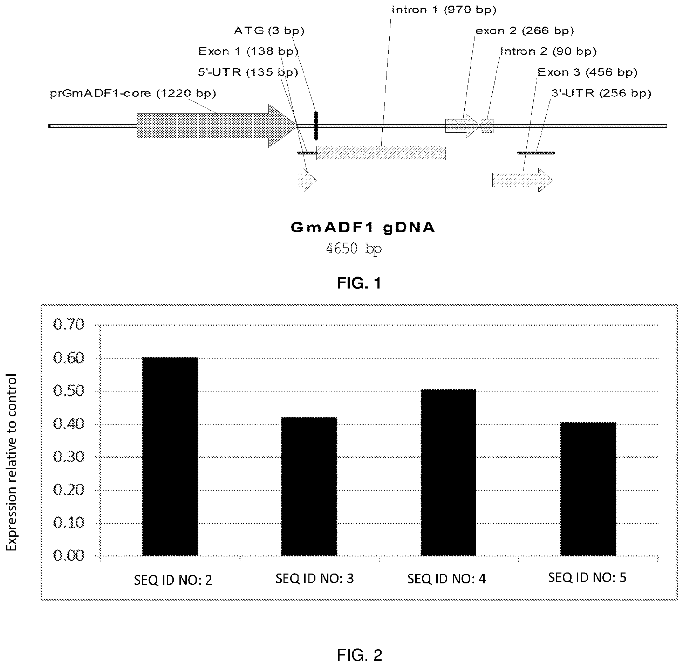

Gene expression analysis was carried out using Affymetrix microarray dataset from the Syngenta Soy Gene Atlas (>50 tissue types) and from published expression profiling experiments using Genevestigator. It was found that dozens of soybean genes are highly expressed throughout the different development stages and in all tested tissues. One of the highly expressed genes encodes *Glyma09g02240*, showing high homology with the ADF1 (ACTIN DEPOLYMERIZING FACTOR 1) family members which are functional in controlling actin-based cellular processes (see, e.g., Staiger et al., 1997, Profilin and actin-depolymerizing factor: modulators of actin organization in plants. Trends in plant Sciences 2: 275-281; Bernstein and Bamburg, 2000, ADF/Cofilin: a functional node in cell biology. Trends in Cell Biology 20: 187-195). The ADFs were first identified in plants and only share 28-35% identity with vertebrate ADF protein sequences. Typically, higher plants have a small family of ADF genes. For example, maize has one constitutive and 2 pollen-specific ADF genes (Lopez et al., PNAS, 93:7415-7420). The gene sequences corresponding to the identified highly expressed GmADF gene were retrieved from soybean variety Williams 82 genome. The predicted gene structure of GmADF1 (prADF1) is shown schematically in Fig. 1.

### Example 2: Identification of active regulatory sequences from GmADF1 promoter by transient expression

In order to identify the active GmADF1 promoter region from Glyma09g02240, different lengths of the GmADF1 upstream regulatory sequences, 5'-UTR region and the 1st intron sequences (prGmADF-01, SEQ ID NO: 1-4) were amplified from the soybean genomic DNA of Williams 82 variety. PCR products were purified and digested with HindIII and BamHI and cloned into a construct to form prGmADF1-ZsGreen-tNOS expression vectors.

To assess the promoter activity of the different prGmADF sequences (SEQ ID NO: 1-*4) in vivo,* the different prGmADF promoter-ZsGreen-tNOS expression cassettes were released from vectors harboring these cassettes by restriction digestion. These prGmADF-ZsGreen-tNOS fragments were then purified and used for bombarding developing soybean cotyledons, which were isolated from soybean seeds of early R7 stage. For each bombardment (using the Bio-Rad PDS-1000/He Biolistic Particle Delivery System), 100 ng of the prCMP-AmCyan-tNOS cassette fragment was used as the loading control on 1µm gold particles. The negative control of the experiment used gold particles loaded only with the control DNA lacking fluorescent protein gene. For GmADF promoter, the amount of prGmADF-ZsGreen-tNOS cassette fragment used was at the equal molar of 200 ng of a prCMP-ZsGreen-tNOS cassette fragment. prCMP-ZsGreen-tNOS cassette serves as the positive control and its fluorescence signal intensity is treated as 100% of activity. Two days after bombardment, CCD images were obtained using Olympus OV100 imaging system for GFP and CFP channels, and the intensity of GFP and CFP signals from transformed cotyledon cells was collected after background subtraction. The intensity of GFP signals on bombarded soybean cotyledons was used as the indicator for GmADF promoter activity. GFP or CFP intensity from 10 most bright cells in each image frame was collected. The average intensity of GFP signals was first normalized by the average intensity of CFP signals from the same experiment, and then compared with the data from prCMP-ZsGreen-tNOS positive control (FIG. 2). Transient expression shows that prGmADF sequences confer excellent promoter activity which is about 40%-60% of the positive control prCMP, which is a strong promoter, in the immature soybean cotyledon tissue (FIG. 2). The 1st intron seems to enhance the promoter activity since the intron-containing sequences SEQ ID NO: 1 and SEQ ID NO: 2 have somewhat higher activity compared to their respective intron-less counterpart SEQ ID NO: 3 and SEQ ID NO: 4.

### Example 3: A modified GmADF promoter sequence, prGmADF-02

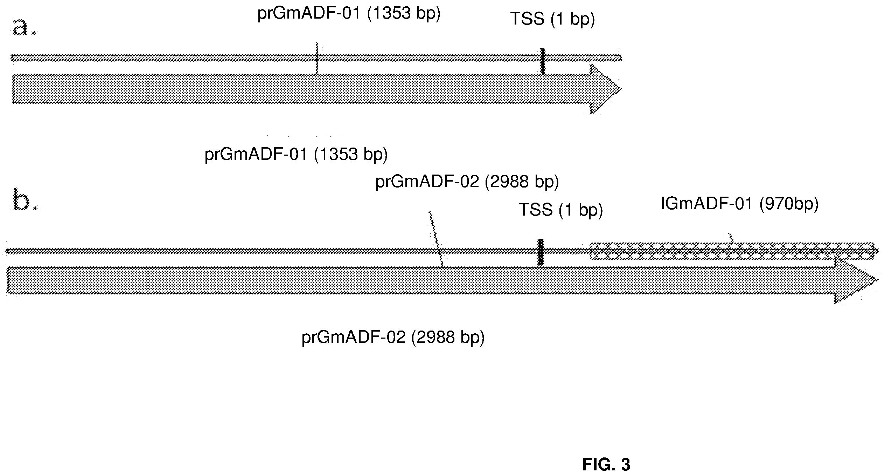

There is a long intron right after the start codon which is present in the exon 1. As discussed above it is believed that the 1st intron is helpful for higher level of gene expression. In order to include this intron as part of the promoter regulatory sequences, an ATG was mutated to a CTG sequence thus removing the start codon. The resulting regulatory sequence pGmADF-02 (SEQ ID NO: 5) is shown schematically in FIG. 3.

prGmADF-02 contains 2 kb upstream of the translation start site plus sequences of exon1, intron1 and 15bp of exon2. For cloning purpose, prGmADF-02 was divided into two parts: P1 and P2 fragments. The BsmBI (compatible with AscI)/BsmBI ("A" in 2001 location was mutated into "C") P1 and the BsmBI (compatible with P1 3' sticky end)/BsmBI (compatible with SpeI) P2 were amplified from soybean (Williams 82) genomic DNA with primers introducing BsmBI in both end, respectively. The P1 and P2 fragments were cut out by BsmBI and purified, then these two fragments were cloned into a backbone (AscI/SpeI digested, and then purified the 9207bp fragment) to form binary vector which contains the prGmADF-02-GUS-tNOS expression cassette and prCMP-PAT-tNOS selectable marker cassette.

### Example 4: Use of regulatory sequences from GmADF1 promoter for driving expression of gene of interest in stable transgenic tobacco and soybean plants

Through site-directed mutagenesis, a SpeI site was introduced into the construct between the KpnI and EcoRI sites after the NOS-terminator. The AmCyan-tNOS fragment was then cut from the resulting plasmid between AgeI and SpeI sites. The second fragment, prGmADF-01 (SEQ ID NO: 4), was cut from a construct using AscI and AgeI. Both fragments, after gel-purification, were cloned into a construct between AscI and SpeI sites by three-fragment ligation to form a binary vector for tobacco and soybean transformation, which contains prGmADF-01-AmCya-tNOS and a hygromycin selectable marker. A binary vector with prADF-02-GUS expression cassette and PAT selectable marker was created as described in Example 3 for tobacco and soybean transformation. The binary vectors were introduced into *Agrobacterium* strain LBA4404 and EHA101 by electroporation. The resulting strains were used for *Agrobacterium-mediated* transformation of tobacco and soybean, respectively. Stable transgenic tobacco and soybean were generated using glufosinate or hygromycin selection as described (see Chilton and Que 2003, Plant Physiol 133:956-965, Hwang et al WO08112044, Que et al WO08112267). Transgenic plants were assayed for reporter gene expression and were found to drive high level expression of AmCyan and GUS expression.

### Example 5: Use of GmADF promoter in driving herbicide tolerance gene in transgenic soybean plants

The prGmADF-01 promoter sequence was PCR amplified from a plasmid with primers introducing BsmBI (compatible with KasI)at 5'- end and BsmBI (compatible with BglII) at 3'- end, then digested by BsmBI, and cloned to another backbone (KasI/ BglII digested). The positive clones were verified by KasI/ BglII digestion. The PCR fragment and both cloning junctions were confirmed by sequencing. The resulting binary vector contained prGmADF-01 driving EPSPS (5-enolpyruvylshikimate-3-phosphate synthase) and was transformed into *Agrobacterium* strain EHA101 and the resulting strain was used to transform soybean variety Jack using glyphosate as selection. Transgenic events were generated from the binary vector using imbibed dry seeds as target tissue as described (Khan US2004034889). Transgenic plants were analyzed by Taqman assay to determine transgene copy number. Table 1 shows that prGmADF-01 promoter driving EPSPS expression resulted in high transformation frequency, similar to a control in which a very strong viral promoter prCMP was used to drive expression of EPSPS gene. This result is surprising considering that in transient expression studies, expression of ZsGreen driven by prGmADF is only 40% to 60% of that by prCMP. Without wishing to be bound by theory, it is possible that prGmADF is more active than prCMP in certain stages of development where prCMP is not very active, such as meristematic tissues where glyphosate is particularly damaging.

**Table 1. Soybean Transformation Efficiency of different promoters driving EPSPS gene using glyphosate selection**

| **EPSPS Selectable Marker Cassette** | **Transformation Frequency** |
|---|---|
| eFMV-prCMP-cmEPSPSpt-tPsE9 | 19.0% |
| eFMV-prGmADF-01-cmEPSPSpt-tPsE9 | 22.19% |

In summary, the above results described in Examples 1-5 indicate that use of soybean ADF regulatory sequences (SEQ ID NO: 1-5) can drive high levels of expression of a gene of interest. The prGmADF promoter also allows for recovery of high transformation efficiency when an herbicide is used as a selection agent indicating a high level of herbicide tolerance.

### Example 6: Cloning and evaluation of soybean promoter candidates using soybean cotyledon transient assay and digital imaging system

### Introduction

In the past, limited options were available to influence the choice of good constitutive promoters for use in generating soybean transgenic events. The main tools available for driving expression of selectable marker and trait genes were Cestrum Leaf Yellow Curling Virus (CmYLCV) promoter (aka. CMP promoter) and several viral enhancers, such as the Cauliflower Mosaic Virus 35S enhancer (e35S), and an enhancer from Figwort Mosaic Virus (FMV). As a consequence, the practice of generating soybean GM products was constrained during the design of binary vectors for transformation. As a result, candidate promoters were identified and tested as described below to identify new promoters that provide constitutive activity.

### Methods

Candidate promoter sequences were amplified from Williams 82 genomic DNA using high fidelity Phusion enzyme (New England Biolabs). The PCR products were digested with BstXI or HindIII at the 5' side, and with AgeI or BamHI at the 3' side before cloned into a cloning vector to swap out prCMP with each candidate promoter. In the resulting constructs, the promoter fragments drove the expression of the ZsGreen gene, making new promoter-ZsGreen-tNOS cassettes for promoter activity tests. All promoter sequences were verified by sequencing before testing.

For the transient assay, the soybean cotyledons were used from seeds of early R7 reproductive stage. Soybean cotyledons were separated from seed pods and placed in a circle on ½ Murashige and Skoog's (MS) medium with the flat side facing up under sterile conditions. A Bio-Rad PDS-1000/He Biolistic Particle Delivery System was used for bombardment. For each experiment, 100 ng of the prCMP-AmCyan-tNOS cassette fragment from a control construct was used as the DNA loading control on 1 µm gold particles. The negative control of the experiment used gold beads loaded with the prCMP-AmCyan-tNOS cassette fragment only. The positive control of the experiment used gold beads loaded with 100 ng of the prCMP-AmCyan-tNOS cassette fragment plus 200 ng of the prCMP-ZsGreen-tNOS cassette fragment from a second control construct. For each promoter, in addition to 100 ng of the prCMP-AmCyan-tNOS cassette fragment, the amount of promoter-ZsGreen-tNOS cassette fragment was loaded at equal molar concentration as 200 ng prCMP-ZsGreen-tNOS cassette fragment. Bombardment was carried out using standard procedures.

Soybean cotyledon pictures were taken and processed as stated in the results (Transient Assay Development section). For quantification, GFP or CFP intensity readings were collected from the 15-20 brightest cells in each picture frame. After elimination of the outliers in the subset detected by the Grubb's test (Grubbs, (1969) Procedures for detecting outlying observations in samples. Technometrics, 11, 1- 21.), measurements from the top ten cells were used to calculate the expression level of each promoter candidate. The average intensity of the GFP signal was normalized by the average intensity of CFP signal from the same picture. The resulted value was then used to compare with the value from the treatment using control prCMP-ZsGreen-tNOS.

Tobacco and soybean transformation experiments were conducted following standard *Agrobacterium-mediated* transformation protocols (see Chilton and Que 2003, Plant Physiol 133:956-965, Hwang et al WO08112044, Que et al WO08112267).

### Results

### Candidate gene selection

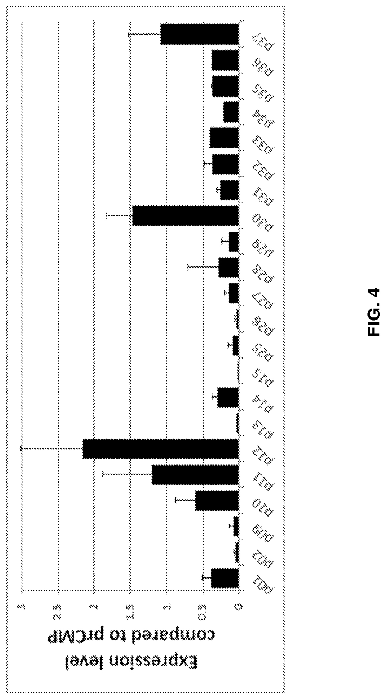

Two searches for strong and constitutive promoter candidates were performed by studying whole genome-based soybean transcriptome dataset generated with Affymetrics Microarray Genechip using soybean plant samples from major organ types across developmental stages. Data was first analyzed from a total of 167 chips covering all tissues and at all stages of soybean development and selected a set of 66 probesets (group 1) that showed a pattern of constitutive expression (average signal greater than 2000 and variance filter 0.02). When plotting Average Gene Expression signal against Standard Deviations (STD) for each candidate, it appeared that although these candidates were expressed at similar levels across all tissues and at all developmental stages, the average gene expression levels of most of the candidates were below 12,000 reads. Since promoters that confer strong expression of a selectable marker gene in younger tissues are more likely to be useful for transformation practice, 66 chips that covered non-root tissues and at early soybean developmental stages were picked out from the 167 chips and used for performing a second search using the same statistical criteria as the first search. From the second search, a subset of 105 probesets (group 2) were identified by 2D-hierarchical clustering analysis that showed a pattern of high constitutive expression (average expression value greater than 12000 reads). Twenty-two genes appeared in both candidate groups. From these groups, three group 1 promoter candidates, eight group 2 promoter candidates, and eleven candidates shared by both groups, were cloned and tested (Fig. 4, Table 2).

**Table 2. List of tested promoter candidates and their corresponding genes**

| Promoter code | SEQ ID NO | Corresponding Gene |
|---|---|---|
| p01 | 6 | Putative plasma membrane intrinsic protein (PIP1) aquaporin. |
| p10 | 7 | Actin depolymerizing factor 1 (ADF1) |
| p11 | 8 | Cyclophilin (Cyp) |
| p12 | 9 | Subi-1 gene for ubiquitin |
| p14 | 10 | No apparent homology |
| p28 | 11 | Glycogen synthase kinase (GSK-3) |
| p30 | 12 | Homologous to elongation factor (EF1) |
| p31 | 13 | Chloroplast photosystem II PsbR |
| p32 | 14 | Glyceraldehyde-3-phosphate dehydrogenase (GAPC) |
| p33 | 15 | Ubiquitin-conjugating enzyme |
| p35 | 16 | Homologous to putative DNA binding protein |
| p36 | 17 | Ubiquitin-conjugation enzyme |
| P37 | 18 | S-adenosylmethionine synthetase (SAMS) |

### Transient assay development and results

A DNA bombardment method was used to deliver test constructs into cotyledons isolated from soybean immature seeds. For the bombardment experiments, a prCMP-AmCyan-tNOS cassette fragment was used as the gene delivery control. The negative control of the experiment used gold beads only loaded with the prCMP-AmCyan-tNOS cassette fragment. The positive control of the experiment used gold beads loaded with prCMP-ZsGreen-tNOS cassette fragment, as the treatment having 100% activity, additional to the prCMP-AmCyan-tNOS cassette fragment.

For all promoter candidates, the promoter fragments were amplified by PCR from genomic DNA of soybean variety Williams 82. The fragments were cloned to drive the expression of a ZsGreen reporter gene. For intron-free candidates, the promoters were ~2kb fragments upstream of the predicted start codon. For candidate genes having one or multiple introns, the corresponding promoter fragments contained ~2kb fragment upstream of the predicted start codon or the first intron, plus components covering the first exon, intron, and 16-18 base pairs of the second exon. Base pairs of the second exon were determined to ensure in-frame fusion of the partial candidate gene product with the reporter ZsGreen gene. Because the fusion proteins contained a six amino acid-linker sequence (Asp-Pro-Pro-Val-Ala-Thr), the activity of the ZsGreen protein was not expected to be significantly affected. All promoter sequences were verified by sequencing before testing. In the tests, the amount of each promoter-ZsGreen-tNOS cassette fragment was loaded at equal molar concentration as the prCMP-ZsGreen-tNOS positive control.

For each promoter candidate, six soybean cotyledons were placed in a circle on ½ Murashige and Skoog's (MS) medium with the flat side facing up for bombardment and imaging. Pictures were taken using Olympus OV100 imaging system two days post bombardment, making sure that each picture included both the soybean cotyledon surface area, and a blank space in the picture as background. For each bombardment experiment, pictures having the most CFP (product of AmCyan expression) spots were chosen for analysis. Background subtraction was applied before choosing spots for quantification. Overlay of CFP and GFP (product of ZsGreen expression) signals suggested that different cotyledon cells received different amounts of AmCyan and ZsGreen reporter genes during bombardment, and therefore could be quantified independently. For quantification, GFP or CFP intensity readings were collected from the 15-20 brightest cells in each picture frame. Measurements from the top ten cells were used to calculate the expression level of each promoter candidate. The average intensity of the GFP signal was normalized by the average intensity of CFP signal from the same picture. The resulting value was then compared to the value from the positive control treatment using prCMP-ZsGreen-tNOS.

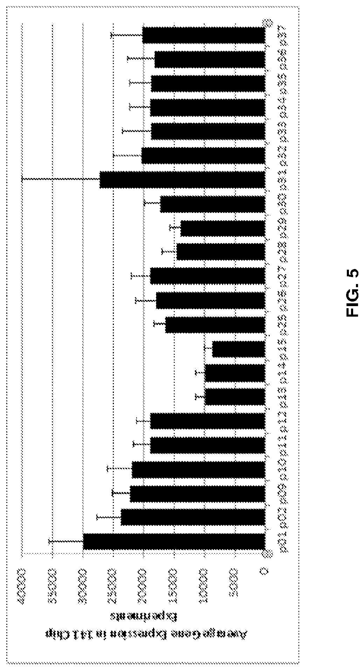

A total of 22 promoter candidates were tested in separate experiments, some were tested three times, and some were tested only once or twice. Because all experiments used prCMP-ZsGreen-tNOS as 100% activity control, results from all these experiments were compiled and presented in one graph (Fig. 5). The promoters p10, p11, p12, p30 and p37 have moderate to strong activity in soybean cotyledons relative to prCMP. The promoters p01, p14, p31, p32, p33, p35, and p36 have lower but reproducibly detectable levels of activities in soybean cotyledons. Table 2 presents genes in the soybean genome driven by these functional promoter candidates. The promoters p2, p9, p13, p15, p26, p27, p28, p29, and p34 did not exhibit detectable, or reproducibly detectable activities, in these transient experiments.

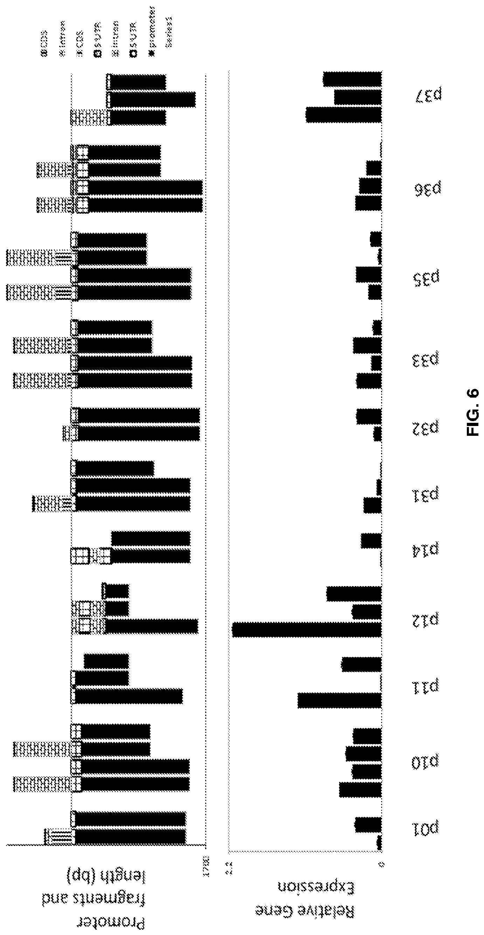

Shorter variants for some of these promoters were also tested using the assay (Fig. 6 and Table 3, the promoter order in Table 3 as read top to bottom is the same as the promoter order in Fig. 6 as read left to right). A correlation between specific introns in promoters and ZsGreen intensity was observed. For instance, introns in promoters p10, p31, p33, p36, and p37 have a positive effect on ZsGreen intensity, whereas introns of p01, p12, p14, p32, and p35 have a negative effect on ZsGreen intensity. These results suggested roles of introns in these promoters in regulating gene expression levels, and helped defining optimal fragment for each promoter to drive the expression of genes of interest.

**Table 3. Promoter sequences and shorter variants**

| Promoter code | SEQ ID NO | Description |
|---|---|---|
| p01 | 6 | Full length |
| p01v2 | 19 | Truncated form with part of 3' sequence removed |
| p01v3 | 42 | Truncated form with part of 5' sequence removed |
| p10 | 7 | Full length |
| p10v2 | 20 | Truncated form with part of 3' sequence removed |
| p10v3 | 21 | Truncated form with part of 5' sequence removed |
| p10v4 | 22 | Truncated form with part of 5' sequence removed and part of 3' sequence removed |
| p11 | 8 | Full length |
| p11v2 | 23 | Truncated form with part of 5' sequence removed |
| p11v3 | 24 | Truncated form with part of 5' sequence removed and part of 3' sequence removed |
| p12 | 9 | Full length |
| p12v2 | 25 | Truncated form with part of 5' sequence removed |
| p12v3 | 26 | Truncated form with part of 5' sequence removed and part of 3' sequence removed |
| p14 | 10 | Full length |
| p14v2 | 27 | Truncated form with part of 3' sequence removed |
| p28 | 11 | Full length |
| p28v2 | 51 | Truncated form with part of 3' sequence removed |
| p28v3 | 52 | Truncated form with part of 5' sequence removed and part of 3' sequence removed |
| p31 | 13 | Full length |
| p31v2 | 28 | Truncated form with part of 3' sequence removed |
| p31v3 | 29 | Truncated form with part of 5' sequence removed and part of 3' sequence removed |
| p32 | 14 | Full length |
| p32v2 | 30 | Truncated form with part of 3' sequence removed |
| p33 | 15 | Full length |
| p33v2 | 31 | Truncated form with part of 3' sequence removed |
| p33v3 | 32 | Truncated form with part of 5' sequence removed |
| p33v4 | 33 | Truncated form with part of 5' sequence removed and part of 3' sequence removed |
| p35 | 16 | Full length |
| p35v2 | 34 | Truncated form with part of 3' sequence removed |
| p35v3 | 35 | Truncated form with part of 5' sequence removed |
| p35v4 | 36 | Truncated form with part of 5' sequence removed and part of 3' sequence removed |
| p36 | 17 | Full length |
| p36v2 | 37 | Truncated form with part of 3' sequence removed |
| p36v3 | 38 | Truncated form with part of 5' sequence removed |
| p36v4 | 39 | Truncated form with part of 5' sequence removed and part of 3' sequence removed |
| P37 | 18 | Full length |
| p37v2 | 40 | Truncated form with part of 3' sequence removed and additional 5' sequence |
| p37v3 | 41 | Truncated form with part of 3' sequence removed |

### Stable Transformation tests and results

Several of these promoters were put into binary vectors to drive the expression of selectable marker genes and were tested in stable transformation experiments. The selectable marker genes and expression elements in these constructs are shown in Table 4.

**Table 4. Binary vectors with expression elements for testing in stable transformation experiments**

| **Construct** | **Enhancers** | | **Promoter** | **Selectable Marker Gene** | **Terminator** |
|---|---|---|---|---|---|
| 19363 | eFMV, e35S, | | prCMP | cmEPSPSpt | tNOS |
| 19437 | eFMV, e35S | | prGmSAMS | cmEPSPSpt | tNOS |
| 19502 | eNOS, eFMV, | | prGmEPSPS | cmEPSPSpt | tGmEPSPS |
| 19501 | eFMV, e35S | | prGmEF (p30) | cmEPSPSpt | tGmEPSPS |
| 19583 | eFMV | | prAtEF1aA1 | cCP4EPSPSCTP2 | tPsE9 |
| 19800 | eFMV, eNtADH | | prCMP | cCP4EPSPSCTP2 | tPsE9 |
| 19809 | | | prGmUbi1 | cCP4EPSPSCTP2 | tPsE9 |
| 19646 | eFMV | | prGmSAMS | cCP4EPSPSCTP2 | tPsE9 |
| 19647 | eFMV | | prGmADF | cCP4EPSPSCTP2 | tPsE9 |
| 19744 | eFMV | | prGmEF (p30) | cCP4EPSPSCTP2 | tPsE9 |
| 19763 | eFMV | | prGmEPSPS | cCP4EPSPSCTP2 | tPsE9 |
| **Key** | | | | | |
| eFMV | | Enhancer from Figwort mosaic virus (FMV) | | | |
| e35S | | Enhancer from Cauliflower mosaic virus (CMV) | | | |
| eNOS | | Putative enhancer sequence | | | |
| eTMV | | Enhancer from Tobacco mosaic virus (TMV) | | | |
| eNtADH | | Enhancer, 5'UTR of tobacco alcohol dehydrogenase gene (ADH). | | | |
| prCMP | | Promoter from Cestrium Yellow Leaf Curling Virus | | | |
| prGmEPSPS | | Promoter from Soybean native EPSPS gene | | | |
| prAtEF1aA1 | | Promoter from *A. thaliana* EF-1 (for elongation factor alpha) A1 gene (prAtTsf1) | | | |
| cmEPSPSpt | | Plastid targeted soybean codon optimized class 2 *A.tumafaciens* EPSPS | | | |
| cCP4EPSPSCTP2 | | | | *Arabidopsis* CTP2 (Chloroplast transit peptide) and CP4EPSPS (Roundup Ready, RR2) fusion. | |
| tNOS | | | | Synthetic Nopaline synthetase terminator | |
| tPsE9 | | | | 3'-UTR of the pea (*Pisum sativum*) rib-1,5-bisphospate carboxylase (rbcS2) small subunit E9. | |
| tGmEPSPS | | | | An EPSPS terminator from *Glycine max* | |

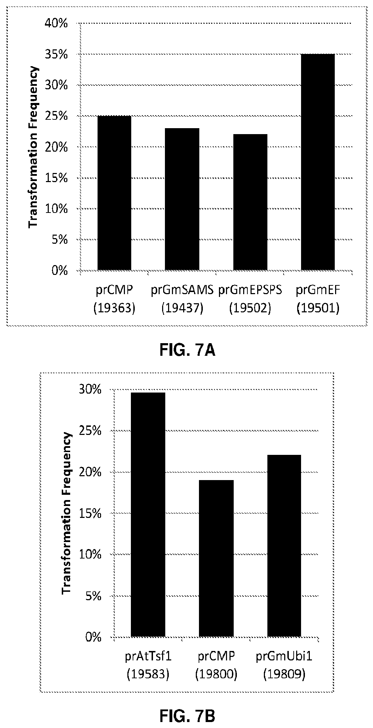

To survive the selection and regeneration processes, selectable marker gene need to be expressed at levels above certain thresh hood in transgenic plants. Therefore, transformation frequency (TF, number of transgenic events/number of explants) in these experiments reflected promoter activities. Constructs 19363, 19437, 19502, and 19501 were tested in tobacco transformation (Fig. 7A). The promoter prGmSAMS (p37 in Fig. 5) exhibited activities comparable to prCMP with an extra enhancer when driving expression of the selectable marker gene EPSPS. The promoter prGmEF (p30 in Fig. 5) exhibited activities higher than that of native EPSPS promoter (prGmEPSPS) with extra enhancers.

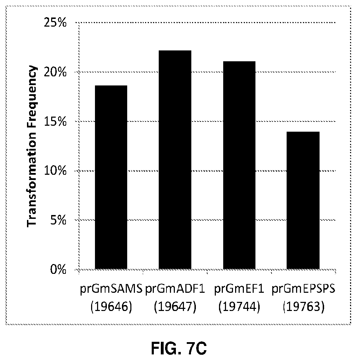

Constructs 19583, 19800, 19809, 19646, 19647, 19744, and 19763 were tested in soybean transformation (Fig. 7B-C). In the absence of enhancer elements, prGmUbi1 (p12 in Fig. 5) did not perform as well as the elongation factor 1-alpha promoter from Arabidopsis thaliana (prAtEF1aA1 aka. prAtTsf1), but was better than prCMP with an extra enhancer, when driving expression of the selectable marker gene CP4EPSPSCTP2 (aka. RR2 for Roundup Ready 2, Fig. 7B). In Fig. 7C, promoters prGmADF1 (p10 in Fig. 5), prGmEF1, and prGmSAMS all exhibited activities higher than prGmEPSPS, when driving the expression of the selectable marker for soybean transformation.

These data from transformation experiments support the transient results, indicating that promoters prGmSAMS, prGmEF, prGmUbi1, and prGmADF are active and strong, efficient in driving selectable marker genes for transformation. Therefore, these promoters are new options that can be used in generating soybean transgenic plants to drive the expression of selectable marker genes and potential trait genes. The other promoters described in this example may also be used to drive expression of selectable marker genes and potential trait genes.

### Example 7: Additional promoter sequences

Additional promoter sequences were developed and are shown in Table 5. These promoters have also been used to drive expression of reporter, selectable marker, or trait genes in stably transformed plants and have been shown to have various levels of expression appropriate for expression of the applicable type of transgene. The strongest performing promoters from Table 5 included prGmADF-03, prGmADF-04, prGmCypCMP-03, and prGmSAMS-03. Accordingly, the promoters in Table 5 may be used to drive expression of, e.g., selectable marker genes and potential trait genes.

**Table 5. Additional promoter sequences**

| **Promoter Name** | **SEQ ID NO** |
|---|---|
| prGmPIP-02 | 43 |
| prGmADF-02 | 44 |
| prGmADF-03 | 45 |
| prGmADF-04 | 46 |
| prGmCyn-02 | 47 |
| prGmCypCMP-01 | 48 |
| prGmCypCMP-02 | 49 |
| prGmCypCMP-03 | 50 |
| prGmSAMS-02 | 53 |
| prGmSAMS-03 | 54 |
| prGmGAPDH2-01 | 55 |
| prGmGAPDH3-01 | 56 |
| prGmGSK3-01 | 57 |

### REFERENCES

US20160237445
WO2007107516
CN103667296B
US6204373

## Claims

1. An expression cassette comprising a nucleotide sequence having at least 90% identity with one or more of SEQ ID NOs: 1-5, 7, 20-22, and 44-46, wherein the nucleotide sequence is operably linked to a heterologous nucleotide sequence.

2. An expression cassette comprising a nucleotide sequence comprising one or more of SEQ ID NOs: 1-5, 7, 20-22 and 44-46, or a biologically active fragment thereof, wherein the nucleotide sequence is operably linked to a heterologous nucleotide sequence.

3. The expression cassette of any one of claims 1 to 2, wherein the heterologous nucleotide sequence is a nucleic acid of interest that encodes an RNA or protein of interest.

4. The expression cassette of claim 3, wherein the RNA or protein of interest is capable of conferring upon a plant a desired characteristic such as antibiotic resistance, virus resistance, insect resistance, disease resistance, resistance to other pests, herbicide tolerance, improved nutritional value, improved performance in an industrial process or altered reproductive capability.

5. The expression cassette of any one of claims 1 to 4, wherein the heterologous nucleotide sequence encodes a selectable marker or wherein expression cassette further comprises a selectable marker.

6. A vector comprising the expression cassette of any one of claims 1 to 5.

7. The vector of claim 6, wherein the vector is a plasmid, virus, or *Agrobacterium* cell.

8. A plant cell comprising the expression cassette or vector of any one of claims 1 to 7.

9. The plant cell of claim 8, wherein the plant cell is a dicot cell, preferably a *Glycine max* cell.

10. A transgenic plant comprising the plant cell of claim 8 or 9.

11. The transgenic plant of claim 10, wherein the plant is a dicot, preferably a *Glycine max* cell.

12. A seed from the transgenic plant of any one of claims 10 to 11, comprising the expression cassette or vector of any one of claims 1 to 7.

13. A method comprising:
i) introducing the expression cassette or vector of any one of claims 1 to 7 into a plant or plant cell through transformation.

14. The method of claim 13, further comprising placing the plant or plant cell under conditions whereby an RNA or protein of interest and/or a selectable marker is expressed from the expression cassette or vector.

15. A transgenic plant, preferably a transgenic dicot plant, more preferably a transgenic *Glycine max* plant, produced by the method of any one of claims 13 to 14, or a plant part thereof comprising the expression cassette or vector of any one of claims 1 to 7.

## Patentansprüche

1. Expressionskassette, umfassend eine Nukleotidsequenz mit mindestens 90 % Identität zu einer oder mehreren der SEQ ID NOs: 1-5, 7, 20-22 und 44-46, wobei die Nukleotidsequenz operativ mit einer heterologen Nukleotidsequenz verknüpft ist.

2. Expressionskassette, umfassend eine Nukleotidsequenz, umfassend eine oder mehrere der SEQ ID NOs: 1-5, 7, 20-22 und 44-46, oder ein biologisch aktives Fragment davon, wobei die Nukleotidsequenz operativ mit einer heterologen Nukleotidsequenz verknüpft ist.

3. Expressionskassette nach einem der Ansprüche 1 bis 2, wobei die heterologe Nukleotidsequenz eine Nukleinsäure von Interesse ist, die eine RNA oder ein Protein von Interesse codiert.

4. Expressionskassette nach Anspruch 3, wobei die RNA oder das Protein von Interesse in der Lage ist, einer Pflanze eine gewünschte Eigenschaft zu verleihen, wie Antibiotikumresistenz, Virusresistenz, Insektenresistenz, Krankheitsresistenz, Resistenz gegen andere Schädlinge, Herbizidtoleranz, verbesserten Nährwert, verbesserte Leistung in einem industriellen Prozess oder verändertes Reproduktionsvermögen.

5. Expressionskassette nach einem der Ansprüche 1 bis 4, wobei die heterologe Nukleotidsequenz einen selektierbaren Marker codiert, oder wobei die Expressionskassette ferner einen selektierbaren Marker umfasst.

6. Vektor, umfassend die Expressionskassette nach einem der Ansprüche 1 bis 5.

7. Vektor nach Anspruch 6, wobei es sich bei dem Vektor um ein Plasmid, Virus oder ein *Agrobacterium-*Zelle handelt.

8. Pflanzenzelle, umfassend die Expressionskassette oder den Vektor nach einem der Ansprüche 1 bis 7.

9. Pflanzenzelle nach Anspruch 8, wobei die Pflanzenzelle eine Dikotylen-Zelle, bevorzugt eine *Glycine-max*-Zelle ist.

10. Transgene Pflanze, umfassend die Pflanzenzelle nach Anspruch 8 oder 9.

11. Transgene Pflanze nach Anspruch 10, wobei es sich bei der Pflanze um eine Dikotyle, bevorzugt eine *Glycine-max*-Zelle, handelt.

12. Samen aus der transgenen Pflanze nach einem der Ansprüche 10 bis 11, umfassend die Expressionskassette oder den Vektor nach einem der Ansprüche 1 bis 7.

13. Verfahren, umfassend:
i) Einbringen der Expressionskassette oder des Vektor nach einem der Ansprüche 1 bis 7 in eine Pflanze oder Pflanzenzelle mittels Transformation.

14. Verfahren nach Anspruch 13, ferner umfassend das Halten der Pflanze oder Pflanzenzelle unter Bedingungen, bei denen eine RNA oder ein Protein von Interesse und/oder ein selektierbarer Marker von der Expressionskassette oder dem Vektor aus exprimiert wird.

15. Transgene Pflanze, vorzugsweise eine transgene Dikotylen-Pflanze, stärker bevorzugt eine transgene *Glycine-max*-Pflanze, produziert durch das Verfahren nach einem der Ansprüche 13 bis 14, oder ein Pflanzenteil davon, umfassend die Expressionskassette oder den Vektor nach einem der Ansprüche 1 bis 7.

## Revendications

1. Cassette d'expression comprenant une séquence nucléotidique ayant au moins 90 % d'identité avec une ou plusieurs des SEQ ID NO : 1 à 5, 7, 20 à 22 et 44 à 46, dans laquelle la séquence nucléotidique est liée de manière fonctionnelle à une séquence nucléotidique hétérologue.

2. Cassette d'expression comprenant une séquence nucléotidique comprenant une ou plusieurs des SEQ ID NO : 1 à 5, 7, 20 à 22 et 44 à 46, ou un fragment biologiquement actif de celles-ci, dans laquelle la séquence nucléotidique est liée de manière fonctionnelle à une séquence nucléotidique hétérologue.

3. Cassette d'expression selon l'une quelconque des revendications 1 à 2, dans laquelle la séquence nucléotidique hétérologue est un acide nucléique d'intérêt qui code pour un ARN ou une protéine d'intérêt.

4. Cassette d'expression selon la revendication 3, dans laquelle l'ARN ou la protéine d'intérêt est capable de conférer à une plante une caractéristique souhaitée telle qu'une résistance aux antibiotiques, une résistance aux virus, une résistance aux insectes, une résistance aux maladies, une résistance à d'autres nuisibles, une tolérance aux herbicides, une valeur nutritionnelle améliorée, des performances améliorées dans un processus industriel ou une capacité reproductrice altérée.

5. Cassette d'expression selon l'une quelconque des revendications 1 à 4, dans laquelle la séquence nucléotidique hétérologue code pour un marqueur sélectionnable ou dans laquelle la cassette d'expression comprend en outre un marqueur sélectionnable.

6. Vecteur comprenant la cassette d'expression selon l'une quelconque des revendications 1 à 5.

7. Vecteur selon la revendication 6, dans lequel le vecteur est un plasmide, un virus ou une cellule d'*Agrobacterium*.

8. Cellule végétale comprenant la cassette d'expression ou le vecteur selon l'une quelconque des revendications 1 à 7.

9. Cellule végétale selon la revendication 8, dans laquelle la cellule végétale est une cellule de dicotylédone, de préférence une cellule de *Glycine max*.

10. Plante transgénique comprenant la cellule végétale selon la revendication 8 ou 9.

11. Plante transgénique selon la revendication 10, dans laquelle la plante est une dicotylédone, de préférence une cellule de *Glycine max*.

12. Semence de la plante transgénique selon l'une quelconque des revendications 10 à 11, comprenant la cassette d'expression ou le vecteur selon l'une quelconque des revendications 1 à 7.

13. Procédé comprenant :
i) l'introduction de la cassette d'expression ou du vecteur selon l'une quelconque des revendications 1 à 7 dans une plante ou une cellule végétale par transformation.

14. Procédé selon la revendication 13, comprenant en outre le placement de la plante ou de la cellule végétale dans des conditions moyennant quoi un ARN ou une protéine d'intérêt et/ou un marqueur sélectionnable est exprimé à partir de la cassette d'expression ou du vecteur.

15. Plante transgénique, de préférence une plante dicotylédone transgénique, plus préférentiellement une plante de *Glycine max* transgénique, produite par le procédé selon l'une quelconque des revendications 13 à 14, ou une partie de plante de celle-ci comprenant la cassette d'expression ou le vecteur selon l'une quelconque des revendications 1 à 7.
